# EUROPEAN PATENT APPLICATION

(11) **EP 1 000 580 A1**
(43) Date of publication of application: **17.05.2000**
(21) Application number: 99117982.1
(22) Date of filing: 15.09.1999
(51) Int. Cl.: A61B 5/05

(54) **Electrical impedance tomography system**

(30) Priority: 11.11.1998 SE 9803862
(71) Applicant: Siemens-Elema AB, 171 95 Solna 1 (SE)
(72) Inventor: Ström, Christer, 941 66 Pitea (SE)

(57) **Abstract**

An electrical impedance tomography system for measuring electrical impedances of an internal body section of a patient (1) is provided. The system includes an electrode array (4a..n) locatable in electrical contact with an external surface of the patient (1) and a data processor (6) for processing the measured electrical impedances and for outputting for display an electrical impedance tomographic representation of the body section dependent thereon. The system further comprises a means (7,9) for providing a physical image illustrative of the body section for use by the data processor (6) in generating for display a superposed combination of the physical image and the representation.

## Description

The present invention relates to an electrical impedance tomography system and in particular to one used in the medical field for generating images of internal body material and structures dependent on their electrical properties.

Monitoring the status of the respiratory system of patients is regularly undertaken in hospitals, especially in intensive care units where patients may be connected to mechanical ventilators for extended periods. Typically airflow at the mouth is measured to provide a continuous non-invasive monitoring of pulmonary mechanical parameters while chest x-ray images provide information on the movement of fluid within the lungs. However, in order to minimise the exposure of the patient to potentially damaging x-ray radiation x-ray images can only be taken sparingly.

Electrical impedance tomography (EIT) is a technique for non-invasively measuring electrical impedances of internal body material and structures, for example the lungs, to provide an electrical impedance image of the body structures of interest. Studies by Alder et al [IEEE Transactions on Biomedical Engineering Vol. 43 No 4, April 1996 pp 414-420] show that EIT may be used to generate images of the conductivity distribution within the lungs in order to monitor lung ventilation, perfusion and fluid content. As no potentially damaging radiation is used to generate the images such EIT monitoring may be carried out continuously throughout the treatment of the patient.

One EIT system that is capable of providing such impedance images of lungs or other internal body structures or body material is described in US 4617939 and comprises typically sixteen or more surface electrodes positionable at known intervals on the surface of patient to define a plane through the body of the patient in which a two-dimensional (2-D) electrical impedance image will be generated. Means are also provided for applying an electrical potential between pairs of electrodes in turn and for measuring the potentials that are induced between all other pairs of electrodes as a consequence of the applied potential. A processing unit and operably connected display unit are provided to process the measured potentials and to display the two-dimensional impedance image of the body section. Modifications to this basic EIT system are disclosed by Metherall et al ["Three-dimensional electrical impedance tomography", Letters to Nature, Nature Vol. 380, 11 April 1996, pp 509-512] which allow three-dimensional impedance images to be constructed and displayed by increasing the number of electrodes and arranging them to define a three-dimensional (3-D) volume.

Unfortunately, the images that are generated by both 2-D and 3-D EIT systems are of relatively low spatial resolution and are often difficult to interpret and to correlate with the physical structure in which the impedances originated.

It is an aim of the present invention to provide an EIT tomography system in which the problem of image interpretation and correlation associated with known devices is at least alleviated.

This is achieved by the system according to the present claim 1. Thus by providing an EIT system which processes a conventionally obtained EIT image and a physical image in order to generate a superposed combination of the two images a visual correlation between the physical structure and the EIT image is made automatically. This also makes the visual interpretation of EIT images more easy. The physical image may be obtained, for example, by computer generation or by using a conventional physical imaging system, such as an x-ray system; a computer aided tomography system; a magnetic resonance imaging system; or an ultrasonic imaging system and may even be a "template image" representative of a typical image of the region of interest.

A conventional physical imaging system may therefore be usefully incorporated into the EIT system and used to generate physical images of the actual patient, thereby enabling a more accurate transposition to be made.

When imaging an internal body structure whose physical properties, such as shape, size or position, may vary cyclically with time, such as will occur for instance with a heart during a cardiac cycle and lungs during a breathing cycle, it is useful to provide a number of different physical image reflecting these variations. An EIT image may then be superposed with one of the physical images selected to be closest in position in the relevant physiological cycle to the EIT image. Alternatively, the EIT image may be generated cyclically at the frequency of the relevant physiological cycle and the physical image taken at the same point in that cycle. In these ways a more accurate interpretation and correlation of a particular EIT image may be made since both the physical image and the EIT image will reflect substantially the same cyclically induced physical variations in the structure or body region of interest.

An embodiment of the present invention will now be described, by way of examples only, with reference to the drawings of the accompanying figures of which

Figure 1 shows a schematic representation of an EIT system according to the present invention.

Figures 2 show a) a stylised representation of an EIT image generated by the system of Figure 1 and b) a stylised representation of a display of a combined image generated by the system of Figure 1.

It is known from US 5,433,198 to provide a visual output of a physical image of the heart onto which is superposed the location of a physical anomaly or the location and/or representation of a catheter. A similar image output modality is also described in WO 96/31753, in which there is also described a computer algorithm for generating the same. Both publications, the contents of which are contained herein by reference, disclose that it is advantageous to superimpose a location of an object within an internal body structure on a physical image of that body structure. However, neither publication describes a system of the present invention in which a superposed combination of a first image of an internal body structure or region, generated using one technique, and a second image of the same body structure or region, generated using a different technique, is provided to aid the interpretation of one or other of the images. US 5,433,198 and WO 96/31753 do however illustrate that algorithms for superposing visual data in order to provide a combined image are known to or may be readily generated by those skilled in the art and are therefore not described in detail herein.

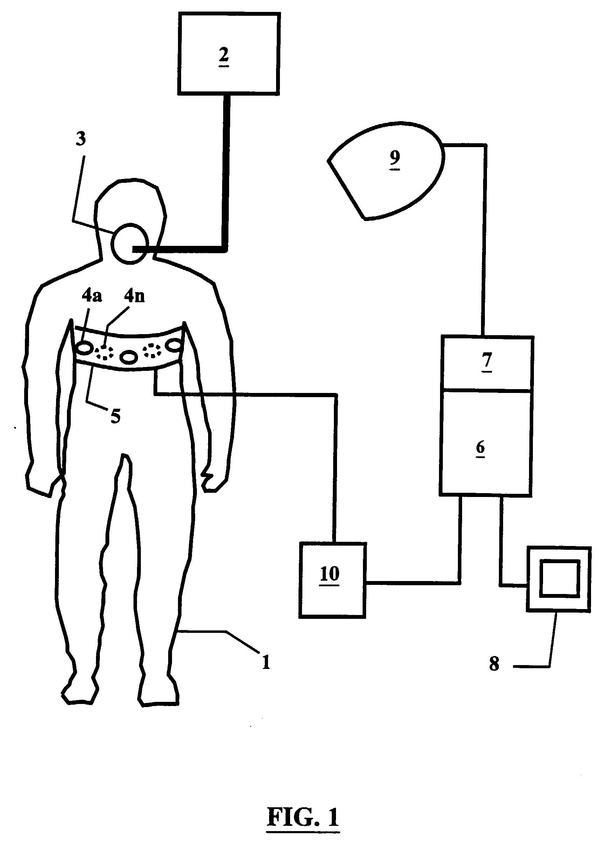

Referring now to Figure 1, a patient 1 is shown connected to a mechanical ventilator 2 via a face mask 3. An EIT system according to the present invention is also connected to the patient 1. This EIT system comprises an array of electrodes 4a..n, typically 16 electrodes, mounted on a belt 5 so as to form a ring about the patient 1 when in use. When the belt 5 is located about the chest of the patient 1, as shown in Figure 1, a tomographic section including the lungs may be generated using the electrode array 4a..n. Also included within the EIT system is a microprocessor unit 6, having integral therewith a digital data store 7; a display unit 8; a conventional X-ray imaging system 9 and an EIT data acquisition system 10. Typically the unit 6 and store 7 can be realised within a conventional personal computer in which its hard disk or its random access memory may be used as the data store 7 dependant largely on whether permanent or temporary storage is required.

The EIT data acquisition system 10 comprises electrical signal generation and measurement electronics which are operably connected to the electrodes 4a..n on the belt 5. The acquisition system 10 is configured to operate in a known manner to successively apply a low amplitude, alternating current across pairs of electrodes and to measure the voltage differences produced between each of the remaining pair combinations formed by the remaining electrodes in the array 4a..n. The measured voltages are passed to the microprocessor unit 6 where an EIT representation, either a so-called "static" or "dynamic" representation, is generated in the known manner to provide a cross sectional EIT image of the lungs. It will be appreciated that the EIT system described may be used to generate an image of various internal body structures, dependent on where the belt 5 is located on the patient 1.

The X-ray imaging system 9, for example a conventional computer aided tomography (CAT) system, is orientated to provide relatively highly detailed X-ray images of the lungs, or relevant part thereof, corresponding to the body section imaged using the electrode array 4a..n. These physical images are digitised and transmitted to the digital data store 7 of the microprocessor unit 6. The images are taken at various known points throughout a breathing cycle of the patient 1 and information on these points are also stored in association with the X-ray images. The information on these points may conveniently be obtained from a control processor (not shown) that is conventionally used to control the operation of the ventilator 2. Although an x-ray imaging system 9 is employed in this embodiment any physical imaging system, such as an ultrasound or a magnetic resonance imaging system may be substituted.

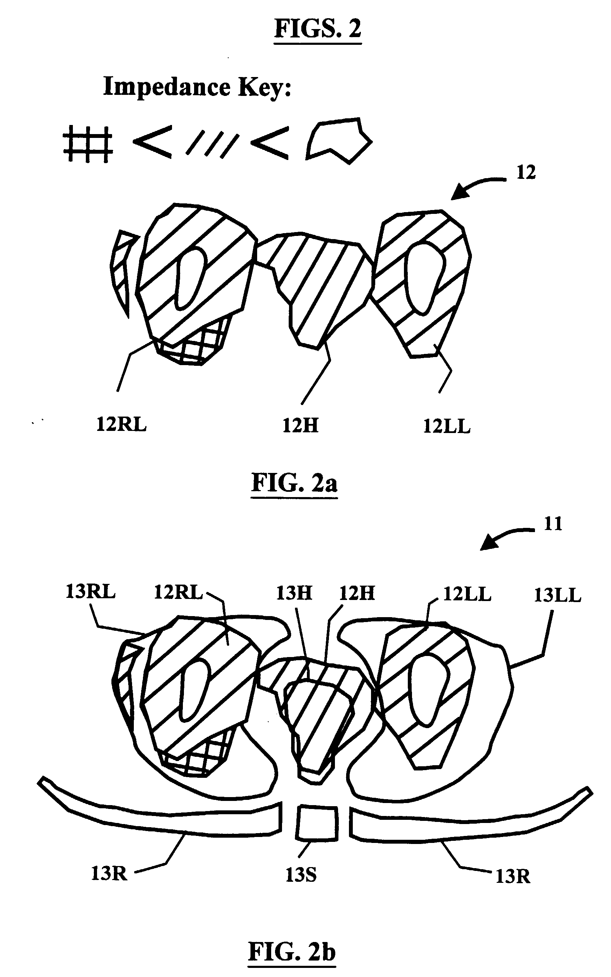

The microprocessor unit 6 is adapted to generate a combined image 11, as shown in Figure 2b, consisting of a transposition of the EIT representation of the lungs 12, which was obtained as previously described at a known point in a patient's breathing cycle (information on this point being obtained from the ventilator 2 in the same manner as described above for the X-ray images), and an X-ray image 13, retrieved from the data store 7 and selected as being the physical image of the lungs closest in time in the breathing cycle to the EIT representation 12.

Figure 2a shows a schematic of an EIT representation 12, generated using the electrode array 4a..n. In this figure, and Figure 2b, the different shading within the EIT image 12 is' used to indicate regions of different impedances as measured using the electrode array 4a..n.

Considering Figure 2a, this image shows the impedance image of the left lung 12LL, the right lung 12RL and of the heart 12H and illustrates that the central portions of each lung are less conductive (higher impedance) than other portions. This indicates that a gas is present within the lungs. Moreover, the areas with higher conductance (lower impedance) typically indicate either an increase in lung tissue (useful in identifying atelectactic areas) or a confluence of liquid in the area. From a functional point of view these areas of higher conductance will not be able to take part in the gas exchange process of the lungs. By monitoring these areas throughout medical intervention (in this embodiment mechanical ventilation using the ventilator 2) the efficacy of the intervention can be reviewed and the intervention modified as necessary.

It is difficult to fully visualise the physical internal body structures that are represented in this EIT image 12 and so correctly interpret the information contained in the EIT image 12. This is particularly true when trying to identify the boundaries between the lungs 12RL,12LL and other body tissue, for example the heart 12H, and also when trying to diagnose the cause of the area of low impedance within the region of the right lung 12RL. However, considering now Figure 2b a superposed combination 11 of an X-ray image 13 and the EIT image 12 is shown. Clearly recognisable X-ray images of the spine 13S and the ribs 13R are shown which helps a user associate the body region illustrated by the combined image 11 with the patient 1. Also shown are X-ray images of the left lung 13LL, right lung 13RL and the heart 13H. It can be seen that the region of low impedance within the right lung 12RL does not lie at the extremity of the lung, towards the spine, as illustrated by the x-ray image 13RL. This would tend to indicate that this low impedance region is probably not caused by liquid (which because of gravity would tend to pool at the lung extremity) but rather is caused by a localised region of collapse. This allows a suitable treatment to be more rapidly chosen, the effect of which may be monitored from changes in the EIT image 12RL.

Preferably the combined image of Figure 2b, generated using the microprocessor 6 and displayed on the unit 8 is colour coded wherein the different shadings of Figure 2b are replaced by specific colours denoting different ranges of impedance values. Indeed, as an alternative, the microprocessor 6 may be programmed to show only those regions of the EIT image where measured electrical impedances lie outside predetermined limits. This may more clearly highlight physical regions of interest and more quickly alert a physician to a risk to a patient's health.

As an alternative the physical images may have been stored in the data store 7 prior to the use of the EIT system and may have been obtained either from the patient 1 or from another subject, in which case the images represent generic "template" images of the lungs. Moreover, a further alternative would be that the microprocessor unit 6 originates its own "computer generated" template images which illustrate the lungs from an algorithm which may readily be constructed by those skilled in the art using a knowledge of human anatomy and of the location of the belt 5 on the patient 1. This last alternative may be useful where it is necessary only to identify, with some degree of certainty, that a certain portion of the EIT representation is probably an image of the lungs or whether it is of some other anatomical feature.

Where the processor unit 6 is to use previously stored physical images or microprocessor generated images then clearly the X-ray imaging system 9 may be omitted.

Furthermore, if the size, shape or position of the body structure which is to be imaged does not alter with time, or if impedance measurements are made (and therefore impedance images generated) at the same point in a relevant physiological cycle, such as the breathing cycle or cardiac cycle, then it will be obvious to those skilled in the art that only one physical image needs to be stored in the digital data store 7.

It will be appreciated by those skilled in the art that although the above embodiment is described in relation to 2-D imaging techniques it may readily be extended to 3-D imaging using known EIT imaging systems and techniques while remaining within the scope of the invention as claimed.

## Claims

1. An electrical impedance tomography system for measuring electrical impedances of an internal body section of a patient (1) comprising an electrode array (4a..n) locatable in electrical contact with an external surface of the patient (1); means (10) for applying an electrical potential between pairs of electrodes and for measuring consequentially generated electrical impedances between other pair combinations of the electrode array (4a..n); and a data processor (6) for processing the measured electrical impedances and for outputting for display a representation (12) of the body section dependent thereon **characterised in that** the system further comprises a means (7,9) for providing a physical image (13) illustrative of the body section for use by the data processor (6) to generate for display a superposed combination (11) of the physical image (13) and the representation (12).

2. A system as claimed in claim 1 **characterised in that** the means for providing a physical image comprises a memory means (7) for digitally storing at least one physical image.

3. A system as claimed in claim 1 or claim 2 **characterised in that** the means for providing a physical image comprises an X-ray imaging system (9) adapted to generate the physical image (13) of the body section of the patient.

4. A system as claimed in claim 1 or claim 2 **characterised in that** the means for providing a physical image (7) is adapted to provide a template image as the physical image.

5. A system as claimed in claim 2 **characterised in that** the memory store (7) is adapted to store a plurality of different physical images obtained at different points in a physiological cycle and in association therewith information on the position of each point and in that the data processor (6) is adapted to select an image from the different physical images for superposing in dependence of the associated position information.
